# NEUE EUROPÄISCHE PATENTSCHRIFT

(11) **EP 1 071 466 B2**
(45) Veröffentlichungstag und Bekanntmachung des Hinweises auf die Entscheidung über den Einspruch: **05.06.2013**
(45) Hinweis auf die Patenterteilung: 16.06.2004
(21) Anmeldenummer: 99919197.6
(22) Anmeldetag: 06.04.1999
(51) Int. Cl.: A61K 47/36, A61K 8/06, A61K 8/73

(54) **GLYCOLIPID-CREMES**
GLYCOLIPID CREAMS
CREMES A BASE DE GLYCOLIPIDE

(30) Priorität: 15.04.1998 DE 19816665
(43) Veröffentlichungstag der Anmeldung: 31.01.2001
(73) Patentinhaber: Henkel AG & Co. KGaA, 40589 Düsseldorf (DE)
(72) Erfinder: FÖRSTER, Thomas, D-40699 Erkrath (DE); WALDMANN-LAUE, Marianne, D-40789 Monheim (DE); MUNK, Gabriele, D-22763 Hamburg (DE); HEINEN, Soraya, D-50935 Köln (DE)
(86) Internationale Anmeldenummer: PCT/EP1999/002322
(87) Internationale Veröffentlichungsnummer: WO 1999/052557

(56) Entgegenhaltungen:
- EP-A- 0 317 313
- EP-A- 0 689 829
- WO-A-96/22073
- WO-A1-98/01109
- DE-A- 2 354 606
- DE-A- 2 514 873
- DE-A- 4 425 268
- US-A- 5 407 678
- PATENT ABSTRACTS OF JAPAN vol. 014, no. 431 (C-0759), 17. September 1990 (1990-09-17) & JP 02 167212 A (SHISEIDO CO LTD), 27. Juni 1990 (1990-06-27)

## Beschreibung

Die Erfindung betrifft kosmetische und pharmazeutische Cremes in Form von Öl-in-Wasser-Emulsionen polarer Ölkomponenten, die zur Stabilisierung lipophile Emulgatoren und wenigstens eine nicht-verdickende modifizierte Stärke enthalten.

Solche Cremes lassen sich ohne die Verwendung üblicher ionischer oder hydrophiler nichtionischer Öl-in-Wasser-Emulgatoren formulieren und weisen daher eine besonders hohe Pflegewirkung auf der Haut auf.

Der Einsatz von Stärkederivaten zur Herstellung kosmetischer Emulsionen ist schon mehrfach beschrieben worden. So wurden z.B. in DE-A-2 354 606 Stärkeester wie z.B. Stärkepropionate als Emulgierhilfsmittel in der Nahrungsmittelindustrie und in der Kosmetik beschrieben. In WO-A-96/22073 sind thermisch inhibierte Stärken beschrieben, die sich auch als verdickende Hilfsmittel in kosmetischen Emulsionen eignen. Aus DE-A-44 25 268 sind emulgatorfreie kosmetische oder dermatologische Öl-in-Wasser-Zubereitungen beschrieben, die anstelle eines Emulgators einen oder mehrere Verdickungsmittel, z.B. auch Polysaccharide enthalten. In diesen Systemen wurde im wesentlichen von den verdickenden Eigenschaften der Stärke oder Stärkederivate zur Stabilisierung der Emulsionen Gebrauch gemacht.

Die vorliegende Erfindung beruht auf der Beobachtung, daß auch nicht-verdickende Stärken. deren wäßrige Lösungen niedrigviskos sind oder die sich aufgrund ihrer vernetzten Struktur in Wasser kaum lösen, in wäßriger Lösung oder Dispersion die Grenzflächenspannung zur angrenzenden Ölphase senken. Es erscheint besonders überraschend, daß dies besonders bei polaren Ölphasen, die bereits eine Grenzflächenspannung unter 30 mN/m zum Wasser aufweisen, noch der Fall ist. Ob dies auf eine Komplexbildung mit bestimmten Ölbestandteilen oder auf eine Konformationsänderung der Polysaccharide in Gegenwart der polaren Öle zurückzuführen ist, ist bisher nicht geklärt. Der Effekt hat uns veranlaßt, solche Emulsionen als Glycolipid-Cremes zu bezeichnen.

Die Aufgabe der vorliegenden Erfindung bestand darin, geeignete Ölkomponenten und Polysaccharide aufzufinden, mit denen die Herstellung von stabilen Öl-in-Wasser-Emulsionen ohne die Mitverwendung ionischer oder hydrophiler nichtionischer Emulgatoren gelingt. Es wurde gefunden, daß nichtquellende, in wäßriger Lösung oder Dispersion dünnflüssige modifizierte Stärken in Kombination mit lipophilen Emulgatoren zu einer sehr dauerhaften Stabilisierung von Öl-in-Wasser-Emulsionen. insbesondere mit polaren Ölkomponenten geeignet sind.

Gegenstand der Erfindung sind kosmetische und pharmazeutische Cremes in Form einer Öl-in-Wasser-Emulsion, deren Ölphase wenigstens eine Ölkomponente enthält, deren Grenzflächenspannung gegen Wasser bei 25°C unterhalb 30 mN/m liegt und die zur Stabilisierung lipophile Emulgatoren mit einem HLB-Wert unter 6 und wenigstens eine nicht-verdickende modifizierte Stärke enthalten, deren 1 Gew.-%ige Lösung oder Dispersion eine Viskosität von weniger als 10 mPa·s bei D = 10/s und 25°C aufweist, und dadurch gekennzeichnet sind dass sie frei sind von ionischen und hydrophilen nichtionischen Emulgatoren mit HLB-Werten von 6 und darüber.

Als kosmetische Cremes werden solche Zusammensetzungen verstanden, die aufgrund der enthaltenen Ölkomponente oder der in der Ölkomponente oder der wäßrigen Phase gelösten Wirkstoffe eine kosmetische Wirkung auf die Haut oder Haare entfalten. So weisen bestimmte Ölkomponenten eine hautweichmachende Wirkung auf. Geeignete öllösliche kosmetische Wirkstoffe sind z.B. Ceramide oder Ceramidanaloga. Vitamine. z.B. Tocopherole oder Tocopherolester, Retinolester, z.B. Retinolpalmitat. Sterine, Bisabolol. Duftstoffe, öllösliche Sonnenschutzmittel, Sebostatika und andere. die sensorischen Eigenschaften der Haut oder der Haare verbessernden oder die Haut oder Haare schützenden Stoffe. Geeignete wasserlösliche kosmetische Wirkstoffe sind z.B. Harnstoff, Allantoin, wasserlösliche Vitamine (Ascorbinsäure), wasserlösliche Sonnenschutzmittel, Proteinderivate, Magnesiumsalze, Rhodanide, Zucker und Polyole wie z.B. Glycerin, Sorbit oder Propylenglycol und wasserlösliche Pflanzenextrakte.

Darüber hinaus können die erfindungsgemäßen Cremes auch öllösliche oder wasserlösliche pharmazeutische oder dermatologische Wirkstoffe zur Behandlung von Krankheiten der Haut oder Kopfhaut enthalten.

Besonders gut eignen sich als Ölkomponenten solche Öle oder Gemische von Ölen, die eine Grenzflächenspannung (γ¹) zu Wasser bei 25° C im Bereich unterhalb von 20 mN/m, bevorzugt, eignen sich als Ölphase ein Öl oder ein Gemisch von Öl- und Fettkomponenten, dessen Grenzflachenspannung zu Wasser bei 25°C unterhalb von 10 mN/m liegt, also Öle mit einer mittleren bis höheren Polarität.

Geeignete Ölkomponenten sind solche mit einer Ester-Gruppe im Molekül, z.B. die Fettsäureester einwertiger C₂-C₁₈-Alkohole wie z.B. Ethylstearat, Isopropylstearat, Isopropylmyristat, Butylstearat, Hexyllaurat oder Stearylisononanoat, auch Dicarbonsäureester wie z.B. Di-n-butyl-adipat oder Diisooctylsuccinat.

Als Ölkomponenten eignen sich auch C₈-C₂₂-Fettsäureester mehrwertiger C₂-C₆-Alkohole, z.B. die als pflanzliche und tierische Öle vorkommenden Fettsäuretriglycerid-Öle, z.B. Olivenöl, Sonnenblumenöl, Sojaöl, Sesamöl, Haselnußöl, Mandelöl, Distelöl. Weniger geeignet ist z.B. Nachtkerzenöl, dessen Polarität so hoch ist, daß es selbst eine beträchtliche Grenzflächenaktivität zeigt. Weiter geeignete Ester dieses Typs sind die Fettsäureester des 1,2-Propylenglycols, des Neopentylglycols, des Trimethylolpropans und des Pentaerythrits.

Bevorzugt sind als Ölkomponenten wenigstens ein Ester eines C₁₂-C₂₂-Alkanols und einer ein- oder mehrwertigen Hydroxycarbonsäure mit 2 - 6 C-Atomen enthalten.

Als C₈-C₂₂-Fettalkoholester einwertiger oder mehrwertiger Hydroxycarbonsäuren sind vor allem die Ester der Glycolsäure, Milchsäure, Äpfelsäure, Weinsäure und Citronensäure geeignet. Solche Ester auf Basis von linearen C_{14/15}-Alkanolen und von in 2-Position verzweigten C_{12/13}-Alkanolen sind unter dem Warenzeichen Cosmacol®-Ester (Hersteller: Eni Chem. Augusta Industriale) von der Fa. Nordmann, Rassmann GmbH & Co, Hamburg, zu beziehen. Diese Ester eignen sich nicht nur besonders gut zur Herstellung der erfindungsgemäßen Glycolipid-Cremes, sondern weisen darüber hinaus auch weitere sehr erwünschte hautkosmetische Wirkungen auf, z.B. beschleunigen sie die Zellerneuerung und verlangsamen die Hautalterung. Die erfindungsgemäßen Cremes können auch Anteile von unpolaren Ölen, z.B. von Paraffinölen oder Silikonölen enthalten, solange die Grenzflächenspannung der Ölkomponenten zum Wasser im Bereich unter 30 mN/m, bevorzugt unter 10 mN/m bleibt.

Die erfindungsgemäßen Cremes enthalten einen lipophilen Emulgator mit einem HLB-Wert unter 6, bevorzugt nichtionische Emulgatoren mit HLB-Werten unter 5. z.B. Fettsäuren oder Fettalkohole mit 12 - 22 C-Atomen oder Partialester von Fettsäuren mit 12 - 22 C-Atomen und Diolen oder Polyolen mit 2 - 10 C-Atomen und 2 - 6 Hydroxylgruppen in einer Menge von 0,1 - 1 Gewichtsteilen pro Gewichtsteil der Ölkomponente.

Der HLB-Wert ist dabei eine Größe, die sich exakt durch die Emulsionsvergleichsmethode bestimmen läßt, in grober Näherung läßt sie sich aus der Beziehung HLB = 0,2 · (100-L) abschätzen, in der L der Anteil der lipophilen Alkyl- oder Acylgruppen in Gew.-% im Emulgatormolekül ist. Solche Emulgatoren werden üblicherweise zur Stabilisierung von Wasser-in-Öl-Emulsionen verwendet. Geeignete C₁₂-C₂₂-Fettalkohole sind z.B. Cetyl- oder Stearylalkohol oder technische Gemische solcher Alkohole.

Geeignete C₁₂-C₂₂-Fettsäurepartialester von C₂-C₆-Polyolen sind z.B. die Mono- und Diester der Palmitin- oder Stearinsäure und des Glycerins oder des Sorbits, z.B. Glycerinmonostearat oder Sorbitansesquistearat.

Andere geeignete lipophile Emulgatoren sind z.B. die Methylglucosid-Fettsäureester oder die Fettsäuremonoethanolamide. Auch freie Fettsäuren eignen sich als lipophile Emulgatorkomponenten.

Als nicht-verdickende modifizierte Stärke wird für die Zwecke der vorliegenden Erfindung eine physikalisch oder chemisch modifizierte Stärke bzw. ein Stärkederivat und zwar entweder ein wasserlösliches Stärkeabbauprodukt oder ein wasserunlöslicher quervernetzter Stärkeester oder Stärkeether eingesetzt. Das wasserlösliche Stärkeabbauprodukt oder der wasserunlösliche quervernetzte Stärkeester oder Stärkeether ist in einer Menge von 1-10 Gew.-% der Creme enthalten. Gemeinsames Merkmal solcher nicht-quellenden Stärken ist die Viskosität einer 1 Gew.-%igen Lösung oder Dispersion in Wasser, die weniger als 10 mPas bei einer Schergeschwindigkeit von D = 10/s und bei 25°C betragen soll.

Während die Verwendung von quellenden bzw. verdickenden Hydrocolloiden als Emulgatoren z.B. aus DE-A 23 54 606 oder aus DE-A- 44 25 268 bekannt war, erschien es überraschend, daß solche nicht-verdickenden Stärken oder Stärkederivate als Emulgatoren die üblichen hydrophilen O/W-Emulgatoren bei der Herstellung stabiler Öl-in-Wasser-Emulsionen ersetzen können, wenn sie zusammen mit lipophilen Emulgatoren zum Einsatz kommen.

Bevorzugt geeignet für die Zwecke der vorliegenden Erfindung sind thermisch und hydrolytisch, ggf. auch enzymatisch abgebaute Stärken, die sich im kalten Wasser klar lösen. Solche Produkte sind im Handel erhältlich, z.B. unter dem Handelsnamen AEROMYL® (Südstärke).

Geeignete Stärke-Derivate sind z.B. oxidativ abgebaute Stärken, z.B. Dialdehydstärken und Dicarboxylstärken und die vemetzten Stärkeester und Stärkeether. Solche, im Handel erhältlichen, weitgehend wasserunlöslichen Produkte sind z.B. das Distärkephosphat, welches z.B. als Mais P04® (Dr. Hauser GmbH.) im Handel ist. Auch quervernetzte Stärkeether z.B. die mit Methylolharnstoff modifizierte "Dimethylimidazolidinone Rice Starch" (gemäß INCI), die unter der Handelsbezeichnung RICE NS (Dr. Hauser GmbH.) erhältlich sind, eignen sich für die Zwecke der Erfindung. Auch hydrophob modifizierte Stärken, z.B. das "Aluminium Starch Octenyl Succinate", eine mit Octenylbernsteinsäureanhydrid modifizierte Stärke, eignet sich für die Zwecke der Erfindung.

Die erfindungsgemäß geeigneten modifizierten Stärken sollen feinteilig sein. Ihre Teilchengröße sollte unter 1 mm für die löslichen Stärken, und die mittlere Teilchengröße der unlöslichen Stärken sollte unter 20 µ liegen.

Durch die erfindungsgemäß geeigneten, nicht-verdickenden modifizierten Stärkeprodukte werden die Öl-in-Wasser-Cremes auch in Abwesenheit von typischen, hydrophilen Öl-in-Wasser-Emulgatoren ausreichend stabilisiert. Es ist daher nicht erforderlich, hydrophile, d.h. wasserlösliche oder ionische Emulgatoren in die Cremes einzusetzen. Die erfindungsgemäßen kosmetischen und pharmazeutischen Cremes sind frei von ionischen und hydrophilen nichtionischen Emulgatoren mit HLB-Werten von 6 und darüber.

Als weitere Hilfsmittel können die erfindungsgemäßen Glycolipid-Cremes übliche galenische Hilfsstoffe in den gebräuchlichen Mengen enthalten. Solche Hilfsmittel sind z.B. wasserlösliche verdickende Polymeren, Schichtsilikate oder pyrogene Kieselsäuren. Weitere gegebenenfalls einzusetzende Hilfsmittel sind z.B. organische Lösungsmittel, z.B. Glycole oder Polyole, Glycerin, 1,2-Propylenglycol, 1,3-Butylenglycol oder Sorbit. Schließlich sollten Konservierungsmittel wie z.B. Pentandiol-1,5, 1,6-Hexandiol, Phenoxyethanol, p-Hydroxybenzoesäureester, Glycin, Sorbinsäure und andere antimikrobielle Komponenten zur Stabilisierung gegen den mikrobiellen Verderb enthalten sein. Schließlich können auch Antioxidantien, Komplexbildner und pH-Puffersubstanzen zur Stabilität von Emulsionen beitragen.

Weitere Hilfsmittel, die in geringen Anteilen in den erfindungsgemäßen Cremes enthalten sein können, sind Duftstoffe, Farbstoffe, Pigmente (z.B. TiO₂-Lichtschutzpigmente) sowie kosmetische und dermatologische Wirkstoffe.

Die folgenden Beispiele sollen den Patentgegenstand näher erläutern:

### Beispiele

### 1. Verwendete Handelsprodukte

### 1.1 Stärkepräparate

| | | |
|---|---|---|
| S1 | Aero-Myl®115 (Südstärke GmbH, D-86522 Schrobenhausen) | |
| | physikalisch modifizierte Kartoffelstärke Viskosität (25°C) der 1 Gew.-%igen Lösung in Wasser | 3,5 mPa s (D=10/s) |
| | | 1,2 mPa s (D=100/s) |
| S2 | Aero-Myl®33 (Südstärke GmbH.) | |
| | physikalisch modifizierte Kartoffelstärke | |
| | Viskosität (25°C) der 1 Gew.-%igen Lösung in Wasser | 1,5 mPa s (D=10/s) |
| | | 1,7 mPa s (D=100/s) |
| | Teilchengrößengröße: mind. 95 % < 1 mm | |
| S3 | Rice NS (Dr. Hauser GmbH, D-82467 Garmisch-Partenkirchen) | |
| | Reisstärke-Methylolharnstoffether | |
| | Viskosität (25°C) der 1 Gew.-%igen Dispersion in Wasser | 1,3 mPa·s (D=10/s) |
| | | 1,2 mPa·s (D=100/s) |
| | Teilchengröße : 0,007 - 0,009 mm | |
| | | |
| S4 | Corn P04 (Dr. Hauser GmbH.) | |
| | Distärkephosphatester, quervernetzt (Basis : Mais) | |
| | Viskosität (25°C) der 1 Gew.-%igen Dispersion in Wasser | 1,3 mPa s (D=10/s) |
| | Mittlere Teilchengröße : 0,02 mm | 1,2 mPa s (D=100/s) |
| | | |
| S5 | Dry-Flo®Plus (National Starch and Chem. Comp.) | |
| | Aluminium-Starch-Octenyl-Succinate | |
| | Viskosität (25°C), 1 Gew.-%ige Dispersion in Wasser | 2,5 mPa s (D=10/s) |
| | | 2,9 mPa s (D=100/s) |

### 1.2 Ölkomponenten

| | |
|---|---|
| Myritol®PC | Propylenglycol-Dicaprylate/Dicaprate |
| Kessco® IPS | Isopropylstearat |
| IPIS | Isopropyl-Isostearat |
| Cetiol®MM | Myristylmyristat |
| Cosmacol®PLG | Gemisch aus Di-C₁₂₋₁₃-Alkyl-Tartrat, Tri-C₁₂₋₁₃-Alkyl-Citrat und Kieselsäure |
| Cetiol®MM | Myristylmyristat |
| Nutralipids®HY1 | Triglycerid-Gemisch aus Safloröl, Aprikosenkemöl, schwarze Johannisbeere-Kernöl und Macadamianußöl |
| Crodamol®PMP | PPG-2 Myristyletherpropionat |
| Baysilonöl®M350 | Polydimethylsiloxan |

| **Zusammengesetzte Ölkomponenten** | | | | |
|---|---|---|---|---|
| | **A** | **B** | **C** | **D** |
| Myritol PC | 25 | 20 | 20 | - |
| Kessco IPS | 50 | 40 | 40 | 100 |
| Cosmacol PLG | 25 | 20 | 20 | - |
| Distelöl | - | 20 | - | - |
| Nachtkerzenöl | - | - | 20 | - |
| Grenzflächenspannung gegen H₂O mN/m (25 ° C) | 7,1 | 10,8 | 4,5 | 27,8 |

### 1.3 Lipophile Emulgatoren

| | |
|---|---|
| Elenor®GMS | Glycerin-mono-/di-palmitat-/stearat aus geh. Rindertalg |
| Stenol®16/18 | Cetyl-/Stearylalkohol |

### 1.4 Wirkstoffe

| | |
|---|---|
| Parsol®1789 | 4-tert.-Butyl-4'-methoxydibenzoylmethan (UV-Filter) |
| Eusolex®6300 | 3(4-Methylbenzyliden)-d,l-campher (UV-Filter) |
| DSH-C | Dimethylsilanol-hyaluronat (wäßrige Lösung) |

### 1.5 Hilfsstoffe

| | |
|---|---|
| Controx KS | Tocopherol-Mono-/diglyceride-Citrat-Gemisch (Antioxidans) |
| Trilon A | Nitrilotriessigsäure-Tri-Na-Salz |
| Sepigel® | Zubereitung von Polyacrylamid, Dodecylpoly-(7EO)-glycolether und Isoparaffin |

### 2. Anwendungsbeispiele

**Tabelle 2**

| | **1** | **2** | **3** | **4** | **5** |
|---|---|---|---|---|---|
| **Ölphase:** | **Gew.-%** | **Gew.-%** | **Gew.-%** | **Gew.-%** | **Gew.-%** |
| Distelöl | 3 | - | 3 | 3 | - |
| Nachtkerzenöl | - | 3 | - | - | - |
| Nutralipids HY-1 | - | - | - | - | 3 |
| Myritol PC | 3,5 | 3,5 | 3,5 | 3,5 | 3,5 |
| Cetiol MM | 2,5 | 2,5 | 2,5 | 2,5 | 2,5 |
| Cutina GMS | 4 | 4 | 2 | 2 | 4 |
| Stenol 16/18 | 2 | 2 | 1,8 | 1,8 | 2 |
| Kessco IPS | 6 | 6 | 6 | 6 | 6 |
| Cosmacol PLG | 3 | 3 | 3 | 3 | 3 |
| Crodamol PMP | 1 | - | 1 | 1 | - |
| Baysilon M350 | - | 1 | - | - | 1 |
| Controx KS | 0,05 | 0,05 | 0,05 | 0,05 | 0,05 |
| Eusolex 6300 | 0,6 | 0,6 | 0,6 | 0,6 | 0,6 |
| Parsol 1789 | 0,1 | 0,1 | 0,1 | 0, | 0,1 |

| **wäßrige Phase:** | **1** | **2** | **3** | **4** | **5** |
|---|---|---|---|---|---|
| | **Gew.-%** | **Gew.-%** | **Gew.-%** | **Gew.-%** | **Gew.-%** |
| Dry Flo Plus | 3 | 3 | - | - | 3 |
| Corn PO4 "B" | - | - | 3 | - | - |
| Rice NS1-070 | - | - | - | 3 | - |
| Wasser dest. | ad 100 | ad 100 | ad 100 | ad 100 | ad 100 |
| 1,3-Butylenglycol | 10 | - | 10 | 10 | - |
| Hydrolite-5 | 1 | - | 1 | 1 | - |
| Trilon A | 0,1 | - | 0,1 | 0,1 | - |
| Glycin | 1 | - | 1 | 1 | - |
| Hexandiol-1,6 | - | 6 | - | - | 6 |
| Dipropylenglycol | - | 5 | - | - | 5 |
| Na-Hyaluronat | 0,01 | 0,01 | 0,01 | 0,01 | 0,01 |
| Wasser dest. | 5 | 5 | 5 | 5 | 5 |
| D.S.H.C. | 5 | 5 | 5 | 5 | 5 |
| Parfümöl | 0,2 | 0,3 | 0,2 | 0,2 | 0,3 |
| Sepigel 305 | - | 0,4 | - | - | 0,4 |
| Green Tea Extract | - | 1 | - | - | 1 |

### Herstellung:

Die Stärkepräparate wurden in der auf 60 - 80°C erwärmten Wasserphase, bestehend aus Wasser, Glycolen, Glycin und wasserlöslichen Hilfsstoffen, unter Verwendung eines Homogenisators (Ultraturax) gelöst (oder dispergiert).

Die auf ca. 80° C erwärmten Ölkomponenten und Emulgatoren wurden langsam und unter fortwährender Homogenisierung mit dem Ultraturax zu der Wasser-Stärke-Phase gegeben und weitere 2 Minuten homogenisiert. Schließlich wurde die Emulsion unter Rühren auf 30° C abgekühlt und die restlichen Komponenten unter Rühren zugegeben.

Es bildeten sich stabile Emulsionen, nach dem Abkühlen auf 20°C glatte Cremes von befriedigender Stabilität.
Die Creme des Beispiels 2 war weniger stabil.

## Patentansprüche

1. Kosmetische oder pharmazeutische Cremes in Form einer Öl-in-Wasser-Emulsion, deren Ölphase
(A) wenigstens eine Ölkomponente, deren Grenzflächenspannung gegen Wasser bei 25°C unterhalb 30 mN/m liegt,
(B) lipophile Emulgatoren mit einem HLB-Wert unter 6 und
(C) zur weiteren Stabilisierung wenigstens eine nicht-verdickende modifizierte Stärke enthält, deren 1 Gew.-%ige Lösung oder Dispersion in Wasser eine Viskosität von weniger als 10 mPa.s (bei einer Schergeschwindigkeit D = 10/s und 25° C) aufweist,
**dadurch gekennzeichnet, dass** sie frei sind von ionischen und hydrophilen nichtionischen Emulgatoren mit HLB-Werten von 6 und darüber.

2. Kosmetische oder pharmazeutische Cremes gemäß Anspruch 1, **dadurch gekennzeichnet, daß** als Ölphase ein Öl oder ein Gemisch von Öl- und Fettkomponenten enthalten ist, dessen Grenzflächenspannung zu Wasser bei 25°C unterhalb von 10 mN/m liegt.

3. Kosmetische oder pharmazeutische Cremes gemäß einem der Ansprüche 1 oder 2,
**dadurch gekennzeichnet, daß** als Ölkomponente wenigstens ein Ester eines C₁₂-C₂₂-Alkanols und einer ein- oder mehrwertigen Hydroxycarbonsäure mit 2 - 6 C-Atomen enthalten ist.

4. Kosmetische oder pharmazeutische Cremes gemäß einem der Ansprüche 1 - 3,
**dadurch gekennzeichnet, daß** als nicht-verdickende modifizierte Stärke ein wasserlösliches Stärkeabbauprodukt oder ein wasserunlöslicher quervernetzter Stärkeester oder Stärkeether in einer Menge von 1 - 10 Gew.-% der Creme enthalten ist.

5. Kosmetische oder pharmazeutische Cremes gemäß Anspruch 1 - 4, **dadurch gekennzeichnet, daß** als lipophile Emulgatoren solche aus der Gruppe der freien Fettsäuren oder Fettalkohole mit 12 - 22 C-Atomen oder der Partialester von Fettsäuren mit 12 - 22 C-Atomen und Diolen oder Polyolen mit 2 - 10 C-Atomen und 2 - 6 Hydroxylgruppen in einer Menge von 0,1 - 1 Gewichtsteilen pro Gewichtsteil der Ölkomponente enthalten sind.

## Claims

1. Cosmetic or pharmaceutical creams in the form of an oil-in-water emulsion of which the oil phase contains
(A)at least one oil component, of which the interfacial tension towards water at 25°C is below 30 mN/m,
(B) lipophilic emulsifiers with an HLB value below 6 and
(C)for further stabilization at least one non-thickening modified starch, the 1 % by weight aqueous solution or aqueous dispersion of which has a viscosity of less than 10 mPa.s at D=10/s and 25°C,
**characterized in that** they are free from ionic and hydrophilic nonionic emulsifiers with HLB values of 6 and higher.

2. Cosmetic or pharmaceutical creams as claimed in claim 1, **characterized in that** an oil or a mixture of oil and fatty components with an interfacial tension to water at 25°C below 10 mN/m is present as the oil phase.

3. Cosmetic or pharmaceutical creams as claimed in claim 1 or 2, **characterized in that** at least one ester of a C₁₂₋₂₂ alkanol and a monobasic or polybasic hydroxycarboxylic acid containing 2 to 6 carbon atoms is present as the oil component.

4. Cosmetic or pharmaceutical creams as claimed in any of claims 1 to 3, **characterized in that** a water-soluble starch degradation product or a water-insoluble crosslinked starch ester or starch ether, is present in a quantity of 1 to 10% by weight of the cream as the non-thickening modified starch.

5. Cosmetic or pharmaceutical creams as claimed in claims 1 to 4, **characterized in that** lipophilic emulsifiers from the group of free fatty acids or fatty alcohols containing 12 to 22 carbon atoms or the partial esters of fatty acids containing 12 to 22 carbon atoms and diols or polyols containing 2 to 10 carbon atoms and 2 to 6 hydroxyl groups are present in a quantity of 0.1 to 1 part by weight per part by weight of the oil component as the lipophilic emulsifiers.

## Revendications

1. Crèmes cosmétiques ou pharmaceutiques sous la forme d'une émulsion huile-dans-eau, dont la phase huileuse contient :
(A) au moins un composant huileux dont la tension superficielle vis-à-vis de l'eau à 25°C se situe en dessous de 30mN/m,
(B) des émulsifiants lipophiles ayant une valeur d'équilibre hydrophilelipophile (EHL) inférieure à 6 et
(C) aux fins de stabilisation plus poussée, au moins un amidon modifié non épaississant dont la solution ou dispersion à 1% en poids dans l'eau présente une viscosité inférieure à 10 mPa.s (à une vitesse de cisaillement D = 10/s et à 25°C),
**caractérisées en ce qu'**elles sont dépourvues d'émulsifiants ioniques et non ioniques hydrophiles avec des valeurs d'EHL égales ou supérieures à 6.

2. Crèmes cosmétiques ou pharmaceutiques selon la revendication 1, **caractérisées en ce qu'**elles contiennent comme phase huileuse une huile ou un mélange de composants huileux et gras dont la tension superficielle vis-à-vis de l'eau à 25°C se situe en dessous de 10 mN/m.

3. Crèmes cosmétiques ou pharmaceutiques selon l'une des revendications 1 ou 2, **caractérisées en ce qu'**elles contiennent comme composant huileux au moins un ester d'un alcanol en C₁₂ à C₂₂ et d'un acide hydroxycarboxylique mono- ou polyvalent comportant de 2 à 6 atomes de carbone.

4. Crèmes cosmétiques ou pharmaceutiques selon l'une des revendications 1 à 3, **caractérisées en ce qu**'elles contiennent comme amidon modifié non épaississant un produit de dégradation d'amidon soluble dans l'eau ou un ester d'amidon ou éther d'amidon réticulé insoluble dans l'eau en une quantité de 1 à 10% en poids de la crème.

5. Crèmes cosmétiques ou pharmaceutiques selon les revendications 1 à 4, **caractérisées en ce qu'**elles contiennent comme émulsifiants lipophiles des émulsifiants du groupe des acides gras ou alcools gras libres comportant de 12 à 22 atomes de carbone ou des esters partiels d'acides gras comportant de 12 à 22 atomes de carbone et de diols ou de polyols comportant de 2 à 10 atomes de carbone et de 2 à 6 groupes hydroxyle en une quantité de 0,1 à 1 partie en poids par partie en poids du composant huileux.
